# EUROPÄISCHE PATENTSCHRIFT

(11) **EP 3 073 944 B1**
(45) Veröffentlichungstag und Bekanntmachung des Hinweises auf die Patenterteilung: **06.09.2017**
(21) Anmeldenummer: 14833116.8
(22) Anmeldetag: 14.11.2014
(51) Int. Cl.: A61B 17/34, A61B 10/02

(54) **NADELFÜHRUNGSVORRICHTUNG FÜR DIE BIOPSIE**
BIOPSY NEEDLE GUIDING DEVICE
DISPOSITIF DE GUIDAGE D'AIGUILLE POUR BIOPSIE

(30) Priorität: 29.11.2013 DE 102013113272
(43) Veröffentlichungstag der Anmeldung: 05.10.2016
(73) Patentinhaber: Noras, Hubert, 97082 Würzburg (DE)
(72) Erfinder: Noras, Hubert, 97082 Würzburg (DE)
(74) Vertreter: Pöhner, Wilfried Anton
(86) Internationale Anmeldenummer: PCT/DE2014/100403
(87) Internationale Veröffentlichungsnummer: WO 2015/078446

(56) Entgegenhaltungen:
- US-A- 5 943 719
- US-A1- 2003 199 754
- US-A1- 2011 257 594
- US-A1- 2013 066 192

## Beschreibung

Die Erfindung betrifft eine Nadelführungsvorrichtung für die Biopsie sowie ein dazugehöriges Verfahren zur deren Verwendung nach dem Oberbegriff der unabhängigen Patentansprüche 1 und 9.

In der Medizin, insbesondere bei der bildgebenden Magnet-Resonanz-Tomographie werden bereits Nadelführungen eingesetzt, um Biopsien vorzunehmen. Der Stand der Technik wird repräsentativ durch EP2347717B1 beschrieben. Die dort beschriebene Biopsienadelführung kann in einer Ebene gedreht werden. In dieser Ebene liegt die Biopsienadel. Üblicherweise steht diese Ebene senkrecht auf der Körperoberfläche, an der die Biopsie durchgeführt werden soll, sodass die Biopsienadelführung verkippt wird. Insbesondere ist diese Ebene nicht senkrecht zur Biopsienadel, denn eine Drehung in dieser Ebene würde lediglich eine triviale axiale Drehung der Biopsienadel bewirken, keine Verkippung. Durch die Verkippung der Biopsienadelführung kann die Biopsie je nach Anwendung und medizinischer Notwendigkeit aus unterschiedlichen Richtungen vorgenommen werden. Eine Steuerung und Veränderung der Biopsierichtung in zwei unabhängigen Ebenen, die nicht senkrecht zur Biopsienadel liegen, ist dabei nicht möglich, sondern nur in einer.

Eine alternative Biopsienadelführung ist in der US20120203095A1 offenbart. Hier ist eine Veränderung der Biopsierichtung in zwei unabhängigen Ebenen, die nicht senkrecht zur Biopsienadel liegen, zwar möglich, doch erfordert dies zwei unabhängige Rotationen, also Rotationen um unterschiedliche Achsen. Zwei Rotationen, die sich nicht auf die Biopsienadel selbst beschränken, sind in Biopsienadelführungen jedoch nachteilig, weil sie mit der üblichen Forderung nach kompaktem Aufbau der gesamten Anordnung kaum in Einklang zu bringen sind. Darüber hinaus ist in US20120203095A1 nur für eine dieser Rotationen eine Skala vorhanden, weshalb die zweite Rotation in der Praxis ungenau gerät, sodass falsche Biopsien passieren können.

Generell gilt, dass die Steuerung und Veränderung der Biopsienadelführung, somit also ihre Verkippung in zwei unabhängigen Ebenen, die nicht senkrecht zur Biopsienadel liegen, nicht durch Rotationsbewegungen des steuernden Elements bewirkt werden sollte. Diese Forderung wird vom Stand der Technik nicht erfüllt.

Der Erfindung liegt deshalb die Aufgabe zugrunde, eine Nadelführungsvorrichtung für die Biopsie zu schaffen, bei der die Biopsierichtung in zwei unabhängigen Ebenen bei präziser Positionierung der Biopsienadel verändert werden kann.

Zur Lösung der Aufgabe schlägt die Erfindung vor, dass eine obere Montageplatte und, dazu parallel, eine untere Montageplatte vorgesehen sind, dass in die obere Montageplatte und die untere Montageplatte eine obere Taumelbuchse bzw. eine untere Taumelbuchse eingelassen ist, dass der Verlauf der Biopsienadel durch die obere Taumelbuchse und die untere Taumelbuchse vorgesehen ist und dass die obere Montageplatte gegenüber der unteren Montageplatte in der Ebene dieser Montageplatten in zwei unabhängigen Richtungen verschiebbar ausgebildet ist. Zwar wird die Biopsienadelführung dabei verkippt, doch dieser Verkippung zugrunde liegen planare, nicht etwa rotatorische Bewegungen der oberen Montageplatte gegenüber der unteren Montageplatte.

Es ist dabei zweckmäßig, auf der der oberen Montageplatte abgewandten Seite der unteren Montageplatte als Halterung eine zur oberen Montageplatte und zur unteren Montageplatte parallele Grundplatte vorzusehen, wobei die untere Montageplatte in der Ebene der Montageplatten in zwei unabhängigen Richtungen gegenüber der Grundplatte verschoben werden kann. Die erfindungsgemäße Verschiebbarkeit der oberen Montageplatte gegenüber der unteren Montageplatte kann ersatzweise ebenso dadurch gewährleistet werden, dass die obere Montageplatte und die untere Montageplatte jeweils in der Ebene der Montageplatten in zwei unabhängigen Richtungen gegenüber der Grundplatte verschoben werden können. Die vektorielle Differenz der Verschiebungen der beiden Montageplatten gegenüber der Grundplatte entspricht dann den Verschiebungen der beiden Montageplatten zueinander.

Vorzugsweise folgen in dieser Reihenfolge aufeinander:
- die Körperoberfläche, an der die Biopsie durchgeführt werden soll,
- die Grundplatte,
- eine erste Montageplatte, im folgenden untere Montageplatte genannt, welche auf der Grundplatte verschiebbar montiert ist, und
- eine zweite Montageplatte, im folgenden obere Montageplatte genannt, welche gegenüber der unteren Montageplatte verschiebbar ist.
Eine Montage der oberen Montageplatte auf der unteren Montageplatte hat den Vorteil, dass die Verschiebung der unteren Montageplatte gegenüber der Grundplatte zu keiner Verschiebung der oberen Montageplatte gegenüber der unteren Montageplatte und demnach auch zu keiner Verkippung der Biopsienadel führt. Das Stecken der Biopsienadel durch die beiden Taumelbuchsen erfolgt vorzugsweise im Inneren einer eigenen Biopsienadelführung, welche anders als die Biopsienadel, die in der Regel für jede Biopsie gewechselt wird, permanent durch die beiden Taumelbuchsen führen kann. Doch auch hier kann eine Auswechselbarkeit der Biopsienadelführung von Nut-

Die Erfindung betrifft eine Nadelführungsvorrichtung für die Biopsie nach dem Oberbegriff des Patentanspruchs 1.

In der Medizin, insbesondere bei der bildgebenden Magnet-Resonanz-Tomographie werden bereits Nadelführungen eingesetzt, um Biopsien vorzunehmen. Der Stand der Technik wird repräsentativ durch EP2347717B1 beschrieben. Die dort beschriebene Biopsienadelführung kann in einer Ebene gedreht werden. In dieser Ebene liegt die Biopsienadel. Üblicherweise steht diese Ebene senkrecht auf der Körperoberfläche, an der die Biopsie durchgeführt werden soll, sodass die Biopsienadelführung verkippt wird. Insbesondere ist diese Ebene nicht senkrecht zur Biopsienadel, denn eine Drehung in dieser Ebene würde lediglich eine triviale axiale Drehung der Biopsienadel bewirken, keine Verkippung. Durch die Verkippung der Biopsienadelführung kann die Biopsie je nach Anwendung und medizinischer Notwendigkeit aus unterschiedlichen Richtungen vorgenommen werden. Eine Steuerung und Veränderung der Biopsierichtung in zwei unabhängigen Ebenen, die nicht senkrecht zur Biopsienadel liegen, ist dabei nicht möglich, sondern nur in einer.

Eine alternative Biopsienadelführung ist in der US20120203095A1 offenbart. Hier ist eine Veränderung der Biopsierichtung in zwei unabhängigen Ebenen, die nicht senkrecht zur Biopsienadel liegen, zwar möglich, doch erfordert dies zwei unabhängige Rotationen, also Rotationen um unterschiedliche Achsen. Zwei Rotationen, die sich nicht auf die Biopsienadel selbst beschränken, sind in Biopsienadelführungen jedoch nachteilig, weil sie mit der üblichen Forderung nach kompaktem Aufbau der gesamten Anordnung kaum in Einklang zu bringen sind. Darüber hinaus ist in US20120203095A1 nur für eine dieser Rotationen eine Skala vorhanden, weshalb die zweite Rotation in der Praxis ungenau gerät, sodass falsche Biopsien passieren können.

Generell gilt, dass die Steuerung und Veränderung der Biopsienadelführung, somit also ihre Verkippung in zwei unabhängigen Ebenen, die nicht senkrecht zur Biopsienadel liegen, nicht durch Rotationsbewegungen des steuernden Elements bewirkt werden sollte. Diese Forderung wird vom Stand der Technik nicht erfüllt.

Aus der US 5,943,719 (Basis für den Oberbegriff des Anspruchs 1) ist eine Nadelführungsvorrichtung für eine Biopsienadel bekannt, die aus zwei übereinander angeordneten Montageplatten bestehen, in die Taumelbuchsen zur Führung der Biopsienadel eingelassen sind. Zweckdienliche bauliche Lösungen zur Fixierung der oberen Montageplatte nach erfolgter relativer Verschiebung finden sich nicht.

Der Erfindung liegt deshalb die Aufgabe zugrunde, eine Nadelführungsvorrichtung für die Biopsie zu schaffen, bei der die Biopsierichtung in zwei unabhängigen Ebenen bei präziser Positionierung der Biopsienadel verändert werden kann.

Zur Lösung der Aufgabe schlägt die Erfindung vor eine Nadelführungsvorrichtung mit den Markmalen den Anspruchs 1 Um einen möglichst kompakten und kostengünstigen Aufbau zu erreichen, ist die obere Montageplatte gegenüber der unteren Montageplatte verschiebbar. zu machen. Arretiert wird sie durch eine zweite Feststellschraube, im folgenden obere Feststellschraube genannt, welche durch eine für die gewünschte Verschiebung der oberen Montageplatte gegenüber der unteren Montageplatte ausreichend groß ausgebildete Öffnung der oberen Montageplatte, im folgenden Montageplattenöffnung genannt, geführt wird. Vorzugsweise folgen aufeinander in dieser Reihenfolge entlang des Schafts der oberen Feststellschraube
- der Kopf der oberen Feststellschraube, welcher als vorzugsweise geriffelte oder gerändelte Kappe ausgebildet ist,
- eine Deckplatte
- die Montageplattenöffnung,
- die untere Montageplatte.
Die obere Feststellschraube führt also nacheinander durch ein Loch der Deckplatte, welche die Montageplattenöffnung zumindest teilweise überlappt, und durch die Montageplattenöffnung, wie gesagt in der oberen Montageplatte. Sie endet in einem Gewindeloch der unteren Montageplatte. Durch Festdrehen der oberen Feststellschraube wird die obere Montageplatte mit Hilfe der Deckplatte auf der unteren Montageplatte festgeklemmt.

Alternativ dazu kann die obere Feststellschraube als in der unteren Montageplatte fest verankerter Schraubbolzen ausgebildet sein. In diesem Fall tritt anstelle des Kopfs der oberen Feststellschraube eine vorzugsweise geriffelte oder gerändelte Kappe mit Innengewinde, die als Mutter für die obere Feststellschraube dient.

Es ist dabei zweckmäßig, auf der der oberen Montageplatte abgewandten Seite der unteren Montageplatte als Halterung eine zur oberen Montageplatte und zur unteren Montageplatte parallele Grundplatte vorzusehen, wobei die untere Montageplatte in der Ebene der Montageplatten in zwei unabhängigen Richtungen gegenüber der Grundplatte verschoben werden kann. Die erfindungsgemäße Verschiebbarkeit der oberen Montageplatte gegenüber der unteren Montageplatte kann ersatzweise ebenso dadurch gewährleistet werden, dass die obere Montageplatte und die untere Montageplatte jeweils Spiel bemessene Führung des Bolzens in der Fräsung der unteren Montageplatte eine Verkippung der Deckplatte verhindern.

Eine Möglichkeit, um Verdrehung der oberen Montageplatte gegenüber der unteren Montageplatte in der Ebene dieser Montageplatten zu verhindern, besteht darin, die untere und die obere Montageplatte in den Bereichen, wo sie aufeinander aufliegen, zu rändeln. Die Rändelungen der beiden Montageplatten müssen zueinander passen. Auf diese Weise lässt sich durch geringfügiges Drehen und anschließendes Einrasten der oberen Montageplatte auf der unteren Montageplatte stets auf einfache Weise diejenige Ausrichtung der oberen Montageplatte gegenüber der unteren Montageplatte finden, bei der die obere Montageplatte gegenüber der unteren Montageplatte nicht verdreht ist. Die nach Festklemmen der beiden Montageplatten aufeinander quantisierte statt kontinuierliche Verschiebung der beiden Montageplatten zueinander kann ohne weiteres hingenommen werden.

Diese Rändelung erfolgt durch Einfräsen paralleler Rillen in zwei verschiedenen, vorzugsweise orthogonalen Richtungen, durch ein abrollbares Rändelwerkzeug, durch eine Fräsmaschine mit einem Stichel, durch eine Stoßmaschine mit einem entsprechend geschliffenen Hobelmeißel, durch eine Kannelierfeile oder in vorteilhafter Weise durch Guss, beispielsweise Spritzguss.

Die Verschiebungen der oberen Montageplatte gegenüber der unteren Montageplatte werden vorzugsweise durch Linierungen der oberen Montageplatte in zwei zueinander senkrechten Richtungen ablesbar gemacht. Diese können in einer Längeneinheit, vorzugsweise Millimeter, oder in einer eine Verkippung der Biopsienadelführung bezeichnenden Winkeleinheit, vorzugsweise Grad, beschriftet sein.

Im letzteren Fall bietet sich zwecks erhöhter Genauigkeit eine äquidistante Skala mit zugehörigen Arkustangenswerten oder einer Tangensskala mit zugehörigen äquidistanten Winkelwerten an. Die Perioden der Rändelung und der Linierung können identisch oder zueinander gebrochen ganzzahlig gewählt werden, was die Einstellung gewünschter Verkippungen erleichtert. Die erforderlichen Verkippungen werden durch Magnetresonanzmessungen vorgegeben.

Zur Unterstützung der vorgenannten Methode zum Verhindern einer Verdrehung der oberen Montageplatte gegenüber der unteren Montageplatte oder als Ersatz dafür ist es zweckmäßig, für jede der Richtungen, entlang derer die obere Montageplatte gegenüber der unteren Montageplatte verschoben wird, zwei identische Linierungen der oberen Montageplatte vorzusehen. Diese werden auf gegenüberliegenden Seiten der Montageplattenöffnung angebracht, durch die die obere Feststellschraube verläuft. Ablesezeiger für die insgesamt vier Linierungen der oberen Montageplatte bilden vier auf der Deckplatte an deren Kanten angebrachte Markierungen. Eine Verdrehung der oberen Montageplatte gegenüber der unteren Montageplatte wird nun verhindert, indem an den gleichen Linierungen auf gegenüberliegenden Seiten der Montageplattenöffnung jeweils gleiche Verschiebungen eingestellt werden.

Um ein Herausfallen einer als eigenes Bauelement ausgeführten Biopsienadelführung aus oberer Taumelbuchse und unterer Taumelbuchse zu vermeiden, kann diese entweder in der oberen Taumelbuchse oder in der unteren Taumelbuchse befestigt sein. Sofern sie nicht aus Hygienegründen, zwecks Sterilisation oder Austausch, demontierbar sein muss, kann sie alternativ dazu Teil entweder der oberen Taumelbuchse oder der unteren Taumelbuchse sein, die auf diese Weise deutlich verlängert wird und zumindest bis in die Ebene weiteren die Verschiebungen der unteren Montageplatte gegenüber der Grundplatte abgelesen bzw. gemessen werden. Die erforderlichen Verschiebungen werden durch Magnetresonanzmessungen vorgegeben.

Dasselbe Konstruktionsschema, nämlich mit Schwalbenschwanzschienen, Feststellschraube, manuellen oder motorischen Zahnstangen-, Spindel- oder ähnlichen Antrieben, kann auch für die Verschiebung der oberen Montageplatte gegenüber der unteren Montageplatte eingesetzt werden.

Um eine Verdrehung der Deckplatte gegenüber der unteren Montageplatte zu verhindern, sodass, wie später beschrieben wird, die Richtung der Biopsienadel den an der oberen Montageplatte und Deckplatte eingestellten Verschiebungen entspricht, kann die Deckplatte mit Führungsbolzen, -schienen oder -profilen versehen sein, die in die untere Montageplatte eingreifen. Beispielsweise kann das Gewindeloch der unteren Montageplatte durch Fräsung in Form eines Quadrats mit gerundeten Ecken angesenkt sein, und in diese Ansenkung als Führung gleitet ein von der Deckplatte ausgehender und mit der Deckplatte fest verbundener Bolzen, dessen Querschnitt ein geringfügig kleineres Quadrat mit gerundeten Ecken ist und der mittig eine Bohrung zur Aufnahme der oberen Feststellschraube aufweist. Bei unzureichender Überlappung von Deckplatte über die Montageplattenöffnung hinaus wird durch diesen Bolzen im Zusammenspiel mit der Feststellschraube außerdem eine Verkippung der Deckplatte gegenüber der unteren Montageplatte vermieden. Falls das nicht ausreicht, kann eine ausreichend tiefe und mit geringem Spiel bemessene Führung des Bolzens in der Fräsung der unteren Montageplatte eine Verkippung der Deckplatte verhindern.

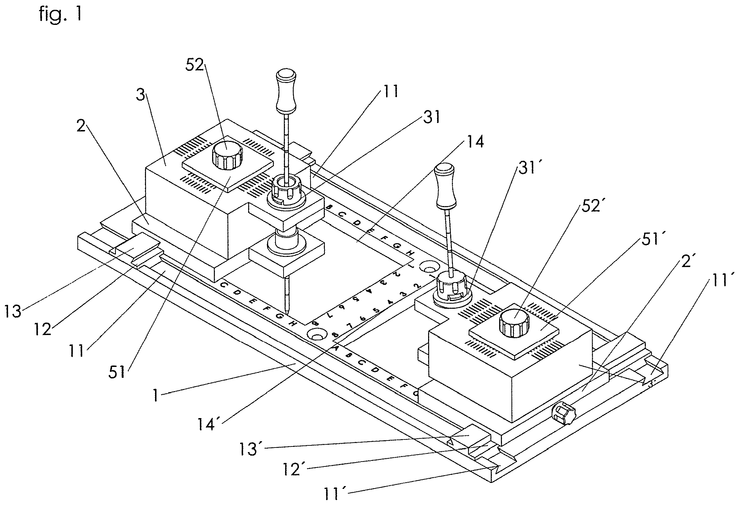

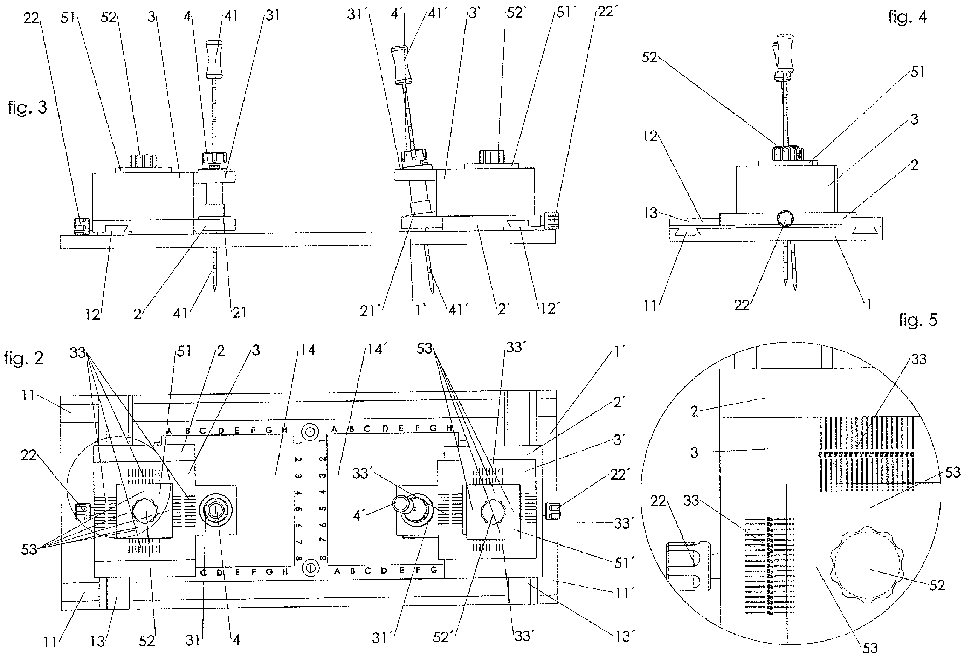

In **Fig. 1** sieht man eine Nadelführungsvorrichtung für Biopsie. In **Fig. 2** ist sie des weiteren von oben, in **Fig. 3** in Queransicht, in **Fig. 4** in Längsansicht zu sehen. Basis und Halterung aller übrigen Bauelemente ist eine Grundplatte 1. Über der Grundplatte befindet sich zunächst eine untere Montageplatte 2, 2', wiederum darüber eine obere Montageplatte 3, 3', Alle diese Platten 1, 2, 2', 3, 3' sind parallel zueinander ausgerichtet und lassen sich entlang ihrer Ebenen zueinander verschieben.

Die Verschiebung zwischen der Grundplatte 1 und der unteren Montageplatte 2, 2' erfolgt über eine erste Schwalbenschwanzverbindung 11, 11', welche eine Verschiebung in einer ersten Richtung zulässt, zu einem Schlitten 13, 13' und von da aus weiter über eine weitere Schwalbenschwanzverbindung 12, 12', welcher eine Verschiebung in einer weiteren, zur ersten Richtung senkrechten Richtung zulässt. Durch Ableseskalen, in **Fig. 1** und **Fig. 2** von 1 bis 8 und von A bis E für die beiden möglichen senkrechten Verschieberichtungen, werden die Verschiebungen der unteren Montageplatte gegenüber der Grundplatte abgelesen. Dies dient zusammen mit den Verkippungen der Biopsienadelführung 4, 4' und dem Abstand zur Körperoberfläche dazu, die Einstichstelle der Biopsienadel 41, 41' festzulegen.

In die untere Montageplatte 2, 2' ist eine untere Taumelbuchse 21, 21' eingelassen. Eine obere Taumelbuchse 31, 31' ist in die obere Montageplatte 3, 3' eingelassen. Deren Taumelachsen - gemeint ist damit die durch die jeweilige Taumelbuchse definierte Achsrichtung - sind aufeinander ausgerichtet und bilden die Achse, entlang derer eine - nur in die **Fig. 3** eingezeichnete - Biopsienadel 41, 41' eingeführt werden kann. Damit die Biopsienadel 41, 41' nicht an der Grundplatte 1 anstößt und obstruiert wird, ist diese mit einer Grundplattenöffnung 14, 14' versehen. Da es in der Regel zu aufwendig wäre, die Taumelachsen von unterer Taumelbuchse 21, 21' und oberer Taumelbuchse 31, 31' zum Einführen der Biopsienadel 41, 41' aufeinander auszurichten, ist als Einsatz in untere Taumelbuchse 21, 21' und obere Taumelbuchse 31, 31' eine eigene Biopsienadelführung 4, 4' vorgesehen. Sie besteht aus einer zur Aufnahme der Biopsienadel 41, 41' geeigneten Röhre, die durch untere Taumelbuchse 21, 21' und obere Taumelbuchse 31, 31' führt. Während sie in der unteren Taumelbuchse 21, 21' frei beweglich ist, ist sie in der oberen Taumelbuchse 31, 31' fixiert. Dies wird durch ein - in den Figuren nicht sichtbares - Innengewinde der oberen Taumelbuchse 31, 31' und dazu passendes Außengewinde der Biopsienadelführung 4, 4' gewährleistet. Um die Verschraubung der Biopsienadelführung 4, 4' in der oberen Taumelbuchse 31, 31' zu erleichtern, ist die Biopsienadelführung 4, 4' da, wo sie auf der von der unteren Montageplatte 2, 2' abgewandten Seite der oberen Montageplatte 3, 3' aus der oberen Taumelbuchse 31, 31' heraussteht, als geriffelter Knopf ausgeführt. Alternativ zu der Fixierung der Biopsienadelführung 4, 4' in der oberen Taumelbuchse 31, 31' durch zu fertigende Gewinde kann die Passung zwischen Biopsienadelführung 4, 4' und oberer Taumelbuchse 31, 31' ausreichend eng ausgeführt werden, sodass ein Durchrutschen verhindert wird. Letzterem Zweck dient dann außerdem ein Kragen, in dem die Biopsienadelführung 4, 4' an der von der unteren Taumelbuchse 21, 21' abgewandten Seite der obere Taumelbuchse 31, 31' endet. Zur Reinigung und Desinfektion kann die Biopsienadelführung 4, 4' ausgebaut werden.

Nach Schieben auf die gewünschte Position wird die untere Montageplatte 2, 2' durch eine untere Feststellschraube 22, 22' in Bezug auf die Grundplatte 1 fixiert. Für die Fixierung der oberen Montageplatte 3, 3' auf der unteren Montageplatte 2, 2' ist eine obere Feststellschraube 52, 52' vorgesehen. Sie besitzt einen geriffelten Kopf und reicht durch eine quadratisch ausgebildete Deckplatte 51, 51', welche zu diesem Zweck in der Mitte ein Loch aufweist, durch eine Montageplattenöffnung 32, 32', welche in der oberen Montageplatte 3, 3' vorhanden ist, bis in die untere Montageplatte 2, 2'. Die Deckplatte 51, 51' besitzt an ihren vier Seiten je eine Markierung 53, 53'. Diese zeigt auf dazu passende Linierungen 33, 33', welche um die Montageplattenöffnung 32, 32' herum auf der der unteren Montageplatte 2, 2' abgewandten Seite der oberen Montageplatte 3, 3' angebracht sind. Genauer ersichtlich ist dies aus der **Fig. 5****,** welche vergrößert das eingekreiste Detail A der **Fig. 2** wiedergibt. Die Linierungen 33, 33' sind in Grad geeicht, wobei es sich hier um Verkippungen der Biopsienadelführung 4, 4' gegenüber einer zur Grundplatte 1 senkrechten Achse handelt. Da die Linierungen 33, 33' linear sind, sind sie nur in der Nähe des Nullpunkts genau, abseits davon dagegen zunehmend ungenau.

Zueinander passende Rändelungen in der unteren Montageplatte 2, 2' und in oberen Montageplatte 3, 3' - nicht sichtbar in den Figuren - können, aber müssen nicht vorgesehen sein.

Die Montageplatten 2, 2', 3, 3' und alle weiteren Bauelemente und Details mit Ausnahme der Grundplatte 1 sind zweifach, also paarig, vorhanden und dabei gegenüber der Mitte der Grundplatte 1 spiegelbildlich angeordnet. Auf diese Weise ergeben sich zwei unabhängige Biopsienadelführungen 4, 4' in der Nähe der Mitte der Grundplatte 1. Dies ist von Vorteil für komplexe Biopsieszenarien.

### Bezugszeichenliste

- 1: Grundplatte
- 11, 11', 12, 12': Schwalbenschwanzverbindung
- 13, 13': Schlitten
- 14, 14': Grundplattenöffnung
- 2, 2': untere Montageplatte
- 21,21': untere Taumelbuchse
- 3, 3': obere Montageplatte
- 31, 31': obere Taumelbuchse
- 32, 32': Montageplattenöffnung
- 33, 33': Linierung
- 4, 4': Biopsienadelführung
- 41, 41': Biopsienadel
- 51, 51': Deckplatte
- 52, 52': obere Feststellschraube
- 53, 53': Markierung

## Patentansprüche

1. Nadelführungsvorrichtung für die Biopsie mit einer Biopsienadelführung (4, 4') für die Einführung einer Biopsienadel (41, 41'), wobei die Biopsienadelführung (4, 4') in einer Ebene, die nicht senkrecht auf der Biopsienadel (41, 41') steht, kippbar ist, **wobei,**
- eine obere Montageplatte (3, 3') und, dazu parallel, eine untere Montageplatte (2, 2') vorgesehen sind,
- in die obere Montageplatte (3, 3') und die untere Montageplatte (2, 2') eine obere Taumelbuchse (31, 31') bzw. eine untere Taumelbuchse (21, 21') eingelassen ist,
- der Verlauf der Biopsienadel (41, 41') durch die obere Taumelbuchse 31, 31' und die untere Taumelbuchse (21, 21') vorgesehen ist und
- die obere Montageplatte (3, 3') gegenüber der unteren Montageplatte (2, 2') in der Ebene dieser Montageplatten (2, 2'), (3, 3') in zwei unabhängigen Richtungen verschiebbar ausgebildet ist, **dadurch gekennzeichnet, dass**
- die obere Montageplatte (3, 3') gegenüber der unteren Montageplatte (2, 2') verschiebbar ausgebildet ist,
- die obere Montageplatte (3, 3') eine Montageplattenöffnung (32, 32') aufweist,
- auf der der unteren Montageplatte (2, 2') abgewandten Seite der oberen Montageplatte (3, 3') eine die Montageplattenöffnung (32, 32') zumindest teilweise überlappende Deckplatte (51, 51') vorgesehen ist,
- zwischen Deckplatte (51, 51') und unterer Montageplatte (2, 2') eine obere Feststellschraube (52, 52') vorgesehen ist, welche durch ein Loch der Deckplatte (51, 51') bis zur unteren Montageplatte (2, 2') verläuft,
- nach einem Festdrehen der oberen Feststellschraube (52, 52') die obere Montageplatte (3, 3') mit Hilfe der Deckplatte (51, 51') auf der unteren Montageplatte (2, 2') festgeklemmt ist.

2. Vorrichtung nach Anspruch 1, **dadurch gekennzeichnet, dass** auf der der oberen Montageplatte (3, 3') abgewandten Seite der unteren Montageplatte (2, 2') als Halterung eine zur oberen Montageplatte (3, 3') und zur unteren Montageplatte (2, 2') parallele Grundplatte (1) vorgesehen ist, wobei die untere Montageplatte (2, 2') in der Ebene dieser Montageplatten (2, 2'), (3, 3') in zwei unabhängigen Richtungen gegenüber der Grundplatte (1) verschiebbar ausgebildet ist und ein unobstruierter Verlauf der Biopsienadel (41, 41') durch die Ebene der Grundplatte (1) vorgesehen ist.

3. Vorrichtung nach Anspruch 1 oder 2, **dadurch gekennzeichnet, dass** zur Verschiebung der unteren Montageplatte (2, 2') gegenüber der Grundplatte (1) eine Schwalbenschwanzverbindung (11), (12), (11'), (12') vorgesehen ist.

4. Vorrichtung nach Anspruch 1 - 3, **dadurch gekennzeichnet, dass** die Deckplatte (51, 51') oder die obere Montageplatte (3, 3') als gegenüber der unteren Montageplatte (2, 2') nicht verdrehbar ausgebildet sind.

5. Vorrichtung nach Anspruch 4, **dadurch gekennzeichnet, dass** die obere Montageplatte (3, 3') und die untere Montageplatte (2, 2') in den Bereichen, wo sie aufeinander aufliegen, zueinander passende Riffelungen aufweisen.

6. Vorrichtung nach Anspruch 1 - 5, **dadurch gekennzeichnet, dass** die Deckplatte (51, 51') und die obere Montageplatte (3, 3') entlang der Kanten der Deckplatte (51, 51') eine Linierung oder Markierung zum Einstellen und Ablesen einer Verschiebung der oberen Montageplatte (3, 3') gegenüber der unteren Montageplatte (2, 2') oder einer Verkippung der Biopsienadel (41, 41') aufweisen.

7. Vorrichtung nach Anspruch 1 - 6, **dadurch gekennzeichnet, dass** auf der Grundplatte (1) bezüglich einer auf der Grundplatte (1) senkrecht stehenden Ebene spiegelverkehrt, also einander gegenüberliegend, jeweils zwei untere Montageplatten (2, 2') mit unteren Taumelbuchsen (21, 21'), obere Montageplatten (3, 3') mit oberen Taumelbuchsen (31, 31') und Biopsienadelführungen (4, 4') vorgesehen sind.

## Claims

1. Needle guiding device for biopsy, comprising a biopsy needle guide (4, 4') for the introduction of a biopsy needle (41, 41'), the biopsy needle guide (4, 4') being tiltable in a plane that does not stand vertically on the biopsy needle (41, 41'), **wherein**
- an upper mounting plate (3, 3') and, parallel thereto, a lower mounting plate (2, 2') are provided,
- into the upper mounting plate (3, 3') and into the lower mounting plate (2, 2') an upper wobble sleeve (31, 31') and a lower wobble sleeve (21, 21') in each case is inset,
- it is provided that the biopsy needle (41, 41') passes through the upper wobble sleeve (31, 31') and the lower wobble sleeve (21, 21') and
- the upper mounting plate (3, 3') is designed so as to be displaceable in two independent directions with respect to the lower mounting plate (2,2') in the plane of these mounting plates (2,2'), (3, 3'), **characterised in that**
- the upper mounting plate (3, 3') is designed so as to be displaceable with respect to the lower mounting plate (2,2'),
- the upper mounting plate (3, 3') has a mounting plate opening (32, 32'),
- on that side of the upper mounting plate (3, 3') that faces away from the lower mounting plate (2,2'), there is provided a cover plate (51, 51') that at least partly overlaps the mounting plate opening (32, 32'),
- between the cover plate (51, 51') and lower mounting plate (2, 2') an upper fixing screw (52, 52') is provided, which passes through a hole of the cover plate (51, 51') as far as the lower mounting plate (2, 2'),
after tightening of the upper fixing screw (52, 52'), the upper mounting plate (3, 3') is clamped firmly on the lower mounting plate (2, 2') with the aid of the cover plate (51, 51').

2. Device according to claim 1, **characterised in that,** on that side of the lower mounting plate (2, 2') that faces away from the upper mounting plate (3, 3') there is provided as holder a base plate (1) which is parallel to the upper mounting plate (3, 3') and to the lower mounting plate (2, 2'), the lower mounting plate (2, 2') being designed such that it is displaceable with respect to the base plate (1) in two independent directions in the plane of these mounting plates (2, 2'), (3, 3') and an obstructed passage of the biopsy needles (41, 41') through the plate of the base plate (1) being provided.

3. Device according to claim 1 or 2, **characterised in that,** for displacement of the lower mounting plate (2, 2') with respect to the base plate (1), a dovetail connection (11), (12), (11'), (12') is provided.

4. Device according to claim 1 to 3, **characterised in that** the cover plate (51, 51') or the upper mounting plate (3, 3') is designed such that it is not rotatable with respect to the lower mounting plate (2, 2').

5. Device according to claim 4, **characterised in that** the upper mounting plate (3, 3') and the lower mounting plate (2, 2') have mutually fitting flutings in the regions at which they lie against one another.

6. Device according to claims 1 to 5, **characterised in that** the cover plate (51, 51') and the upper mounting plate (3, 3') have, along the edges of the cover plate (51, 51'), a ruling or marking for adjusting and reading off of a displacement of the upper mounting plate (3, 3') with respect to the lower mounting plate (2, 2'), or of a tilting of the biopsy needle (41, 41').

7. Device according to claims 1 to 6, **characterised in that,** on the base plate (1), with respect to a plane that stands vertically on the base plate (1), in mirror image, that is to say opposite one another, there are provided, in each case, two lower mounting plates (2, 2') with lower wobble sleeves (21, 21'), upper mounting plates (3, 3') with upper wobble sleeves (31, 31') and biopsy needle guides (4, 4').

## Revendications

1. Dispositif de guidage d'aiguille pour la biopsie avec un guidage d'aiguille à biopsie (4, 4') destiné à l'introduction d'une aiguille à biopsie (41, 41'), sachant que le guidage d'aiguille à biopsie (4, 4') peut être basculé dans un plan qui n'est pas orienté à la verticale par rapport à l'aiguille à biopsie (41, 41'), **sachant que**
- une plaque de montage supérieure (3, 3') et une plaque de montage inférieure (2, 2') parallèle à la première sont prévues,
- une douille oscillante supérieure (31, 31') est insérée dans la plaque de montage supérieure (3, 3') et une douille oscillante inférieure (21, 21') est insérée dans la plaque de montage inférieure (2, 2'),
- le parcours de l'aiguille à biopsie (41, 41') est prévu par la
- douille oscillante supérieure (31, 31') et la douille oscillante inférieure (21, 21'),
- la plaque de montage supérieure (3, 3') étant réalisée en face de la plaque de montage inférieure (2, 2') dans le plan de ces plaques de montage (2, 2'), (3, 3') de façon à pouvoir glisser dans deux directions indépendantes, **caractérisé par le fait que**
- la plaque de montage supérieure (3, 3') est réalisée en face de la plaque de montage inférieure (2, 2') de façon à pouvoir glisser,
- la plaque de montage supérieure (3, 3') présente une ouverture de plaque de montage (32, 32'),
- une plaque de recouvrement (51, 51') dépassant au moins en partie l'ouverture de la plaque de montage (32, 32') est prévue sur le côté de la plaque de montage supérieure (3, 3') opposé à la plaque de montage inférieure (2, 2'),
- une vis d'arrêt supérieure (52, 52') est prévue entre la plaque de recouvrement (51, 51') et la plaque de montage inférieure (2, 2'), laquelle passe à travers un orifice dans la plaque de recouvrement (51, 51') jusqu'à la plaque de montage inférieure (2, 2'),
- la plaque de montage supérieure (3, 3') étant solidement serrée sur la plaque de montage inférieure (2, 2') à l'aide de la plaque de recouvrement (51, 51') après que la vis d'arrêt supérieure (52, 52') a été vissée à fond.

2. Dispositif selon la revendication 1, **caractérisé par le fait qu'**une plaque de base (1) parallèle à la plaque de montage supérieure (3, 3') et à la plaque de montage inférieure (2, 2') est prévue en tant que fixation sur le côté de la plaque de montage inférieure (2, 2') opposé à la plaque de montage supérieure (3, 3'), sachant que la plaque de montage inférieure (2, 2') est réalisée de façon à pouvoir glisser vis-à-vis de la plaque de base (1) dans deux directions sur le niveau de ces plaques de montage (2, 2'), (3, 3') et qu'un parcours sans obstruction de l'aiguille à biopsie (41, 41') est prévu à travers le plan de la plaque de base (1).

3. Dispositif selon la revendication 1 ou 2, **caractérisé par le fait qu'**une liaison en queue d'aronde (1), (12), (11'), (12') est prévue pour faire glisser la plaque de montage inférieure (2, 2') par rapport à la plaque de base (1).

4. Dispositif selon la revendication 1 à 3, **caractérisé par le fait que** la plaque de recouvrement (51, 51') ou la plaque de montage supérieure (3, 3') est réalisée de façon à ne pas pouvoir tourner par rapport à la plaque de montage inférieure (2, 2').

5. Dispositif selon la revendication 4, **caractérisé par le fait que** la plaque de montage supérieure (3, 3') et la plaque de montage inférieure (2, 2') présentent des cannelures adaptées les unes aux autres dans les zones où elles reposent l'une sur l'autre.

6. Dispositif selon la revendication 1 à 5, **caractérisé par le fait que** la plaque de recouvrement (51, 51') et la plaque de montage supérieure (3, 3') présentent, le long des bords de la plaque de recouvrement (51, 51'), un lignage ou un marquage pour régler et lire un décalage de la plaque de montage supérieure (3, 3') par rapport à la plaque de montage inférieure (2, 2') ou un basculement de l'aiguille à biopsie (41, 41').

7. Dispositif selon la revendication 1 à 6, **caractérisé par le fait que** deux plaques de montage inférieures (2, 2') avec des douilles oscillantes inférieures (21, 21'), des plaques de montage supérieures (3, 3') avec des douilles oscillantes supérieures (31, 31') et des guidages d'aiguilles à biopsie (4, 4') sont prévues sur la plaque de base (1), en miroir par rapport à un niveau disposé à la verticale de la plaque de base (1), c'est-à-dire l'une en face de l'autre.
